# EUROPEAN PATENT SPECIFICATION

(11) **EP 3 049 015 B1**
(45) Date of publication and mention of the grant of the patent: **10.03.2021**
(21) Application number: 13777348.7
(22) Date of filing: 23.09.2013
(51) Int. Cl.: A61B 90/00, A61B 90/11, A61B 90/50, A61B 90/10, A61B 34/10

(54) **SYSTEM AND METHOD FOR ALIGNING A MEDICAL DEVICE**
SYSTEM UND VERFAHREN ZUR AUSRICHTUNG EINER MEDIZINISCHEN VORRICHTUNG
SYSTÈME ET PROCÉDÉ D'ALIGNEMENT D'UN DISPOSITIF MÉDICAL

(43) Date of publication of application: 03.08.2016
(73) Proprietor: Demcon Advanced Mechatronics B.V., 7521 PH Enschede (NL)
(72) Inventor: ARNOLLI, Maarten, NL-7513 KD Enschede (NL); FRANKEN, Michel, NL-7511 GH Enschede (NL); BUIJZE, Martijn, NL-7555 KV Hengelo (NL)
(74) Representative: Luten, Martin Haaije
(86) International application number: PCT/NL2013/050679
(87) International publication number: WO 2015/041516

(56) References cited:
- US-A1- 2003 109 825
- US-A1- 2006 036 264
- US-A1- 2009 112 084
- US-A1- 2009 234 369
- US-A1- 2010 198 052
- US-A1- 2013 066 232

## Description

The present invention relates to a system for aligning a medical device with respect to a patient with an insertion axis. The invention further relates to a method for aligning a medical device with respect to a patient.

For certain medical operations, such as a biopsy or percutaneous ablation, it is necessary to accurately insert an elongated medical device, such as a needle, into a patient along a predetermined trajectory towards the target tissue. This trajectory is for instance planned preoperatively based on medical scans of the patient such that the target tissue can be reached with minimal damage in surrounding tissue. Based on this trajectory, an entry location and a predetermined insertion orientation along an insertion axis, in terms of insertion angles, on the patient are determined.

Accurately aiming the needle with respect to the predetermined entry location on the patient during an intervention has proven to be difficult and time inefficient. It is for instance known to partially insert the needle into the patient by hand in the predetermined location along an axis globally corresponding to the predetermined insertion axis. The position and orientation of the needle are subsequently determined using a medical imaging device, such as a CT-scan or MRI-scanner. Based on this detected orientation, the position of the needle is adjusted manually towards the predetermined insertion axis. These steps are repeated until the needle is in alignment with the predetermined insertion axis.

It is known to use a system for aligning medical devices which are arranged to hold and align a medical device with respect to the patient. Instead of manually moving the needle towards the predetermined orientation after each imaging step, the system mechanically moves the needle. The steps of detecting the orientation of the needle by imaging and moving the needle based on the detected orientation are again iteratively repeated until the actual detected orientation corresponds to the predetermined orientation.

US 2010/0198052 A1 describes a lockable articulating arm onto which a trajectory guide frame can be secured.

US 2009/0112084 describes such a trajectory guide frame.

It is however a drawback of these known systems that aligning the medical device, such as a needle, still takes a considerable amount of time and involves repeatedly imaging the position and/or orientation of the medical device with respect to the patient.

It is therefore a goal of the present invention, amongst other goals, to provide an reliable and/or efficient system for aligning a medical device with respect to a patient.

This goal, amongst other goals, is met by system according to claim 1.

According to the invention, the position and orientation of the stationary part of the alignment device are detected once using at least one fiducial marker. The position of the fiducial marker, or preferably a plurality thereof, with respect to the stationary device is thereto known, such that after determining the position and orientation of the fiducial marker(s) using a medical imaging device such as a CT-scanner or MRI-scanner, the position and orientation of the stationary part of the alignment device are also known. Based on this determination, the alignment device can be automatically operated, for instance by a suitable controller, to align the axial guide such that a medical device, inserted or to be inserted into the axial guide, is properly aligned with the insertion axis. The fiducial marker is therefore arranged for determining the position and orientation of the stationary part of the alignment device as a reference for rotating the axial guide for the medical device to a predetermined orientation in which the guiding axis of the axial guide is aligned with the insertion axis. As the position of the remote centre of rotation with respect to the stationary part is fixed, the guiding axis can be aligned accurately by rotating the axial guide about said remote centre of rotation.

The axial guide is arranged to guide, and optionally hold, the medical device such that a longitudinal axis, i.e. the guiding axis, of the medical device held or to be held intersects the remote centre of rotation. The alignment device is preferably arranged to rotate the axial guide along at least a part of a spherical surface having the remote centre of rotation as a centre. The alignment device is then arranged to rotate the medical device, if held in the axial guide, without changing the distance between the axial guide, and thereby the medical device, and the remote centre of rotation, and thereby the patient. Accidentally inserting the medical device upon rotation is thus prevented if the axial guide is rotated when the medical device is held by the axial guide. It is however preferred to first rotate the axial guide such that the guiding axis as defined by the axial guide is in alignment with the insertion axis before inserting the medical device into the axial guide.

The axial guide preferably comprises a receptacle for receiving the medical device such that any movement other than along the longitudinal axis, i.e. the guiding axis, is prevented. The guiding axis is thereby uniquely defined by the axial guide. In other words; it is preferred if the medical device can only be inserted into the axial guide in a single orientation. Any movement along the guiding axis, when the medical device is properly aligned with the insertion axis, will then result in a movement of the medical device along this insertion axis.

Although it is possible to position the remote centre of rotation using suitably driven motors for moving the stationary part of the alignment device, it is preferred if the stationary part is positioned manually such that the remote centre of rotation is located on the location, i.e. the point of incision, by hand on the patient. Therefore, according to a preferred embodiment, the system hereto comprises a handle associated with the stationary part of the alignment device for manually positioning the remote centre of rotation on the location on the patient. Preferably, the handle comprises at least one fiducial marker to allow a reliable determination of the position and orientation of the stationary part.

It is preferred if the locking means are arranged to allow manual, i.e. passive, movement between the base and the stationary part in the moveable position. By not actively moving the stationary part with respect to the base, for instance using driving means, a simple and efficient configuration of the system is obtained. The locking means thereto for instance comprise friction means arranged to impose a friction force between the moveable parts of the arm in the moveable position. The locking means may limit relative movement between the stationary part and the base if the exerted force on the stationary part is smaller than the friction force and which can be overcome by manually moving the stationary part.

To allow easy handling of the stationary part of the alignment device, it is preferred if the stationary part of the alignment device extends at a middle part of the handle, wherein the handle extends at both sides of the stationary part. The user can grip the handle at both sides of the stationary part such that the remote centre of rotation can be steered to the predetermined location on the patient. The handle and stationary part preferably form a robust and rigid combination. It is further preferred if the arm is connected to the combination of the stationary part and handle at the middle part. More preferably, to allow an accurate determination of the position and orientation of the stationary part, both sides of handle are provided with fiducial makers, while also at least one fiducial marker is provided on a central location.

To allow the positioning of the remote centre of rotation on the predetermined location on the patient, the arm is moveable between a moveable position for moving the alignment device with respect to the base and a locked position wherein the relative position between the base and the alignment device, in particularly the stationary part thereof, is fixed. In this locked position, the position and orientation of the stationary part is therefore fixed with respect to the surroundings, as the base is connected thereto.

It is remarked that the position of the remote centre of rotation with respect to the stationary part of the alignment device is fixed, regardless of the orientation and position of the axial guide with respect to this stationary part. The position of the axial guide with respect to the stationary part is therefore not critical when the remote centre of rotation is placed, preferably manually, on the patient. As the alignment device is arranged to move the axial guide for aligning the guiding axis with the insertion axis after initial placement of the remote centre of rotation, also the orientation of the stationary part is not critical when placing the remote centre of rotation on the patient.

Although it is possible that the insertion axis and the location on the patient are determined pre-operatively, it is preferred if the system is arranged to compensate for deviations in placement of the remote centre of rotation on the patient, since it is in practice not always possible or time efficient to place the remote centre of rotation exactly on the predetermined location on the patient. Generally, it is preferred if the insertion axis intersects, or extends through, the remote centre of rotation and the position of the target location in the patient. Therefore, a further preferred embodiment of the system comprises a controller arranged for determining the position of the remote centre of rotation on the patient based on the determined position and orientation of the stationary part. Since the relative position of the remote centre of rotation is fixed with respect to the stationary part, the position of the remote centre of rotation can be determined based on the already determined position of the stationary part. The controller is then further arranged to determine the insertion axis based on the position of the target location in the patient and the position of the remote centre of rotation such that the insertion axis intersect the remote centre of rotation and the target location in the patient. The insertion axis is (re)calculated based on the actual location of the remote centre of rotation on the patient, such that this calculated insertion axis actually intersects both the remote centre of rotation, which preferably corresponds to the insertion location, and the target location in the patient. The alignment device can then be used to align the guiding axis of the axial guide with this insertion axis.

The controller is preferably arranged to determine, or receive, the position of the target location in the patient using medical imaging. The associated scanner hereto images, next to the fiducial for determining the position and orientation of the stationary part, also a part of the patient including the target location. It is for instance possible that the user of the system indicates the target location in an obtained image, wherein this position is subsequently provided to the controller for (re)calculating the insertion axis.

It is then for instance possible that an initial location on the patient and an associated insertion axis which intersects this initial location and the target location are determined pre-operatively, and that after globally placing the remote centre on the patient near the initial location, the insertion axis is (re)calculated based on the actual location of placement. It is hereby preferred if the system is arranged to provide output, for instance to a screen, concerning the new insertion axis, such that the user of the system can confirm that also the recalculated insertion axis does not intersect any vital organs in the patient.

To provide sufficient degrees of freedom for positioning the remote centre of rotation on the patient such that the relative position between the system and the patient is less critical, the arm preferably comprises a plurality of arm sections interconnected by lockable ball joints. More preferably, the arm comprises two lockable ball joint, one near the base and one near the stationary part. Lockable ball joints as such are known in the art. Preferably, the system comprises an input device, for instance a button, and an associated actuator for moving the locking means between the moveable and locked positions. It is for instance possible that the input device is located on the handle, such that the arm can be moved between the positions while operating the handle. Suitable transfer means are then arranged between the input device and the locking means.

According to a further preferred embodiment, the alignment device comprises a first arm and a second arm comprising the axial guide, wherein the first arm is rotatably connected to the stationary part, wherein the first arm is rotatable with respect to the stationary part and wherein the second arm is rotatable with respect to the first arm for rotating the axial guide about the remote centre of rotation along at least part of a spherical surface.

Preferably, the first arm is rotatable with respect to the stationary part about a first axis which intersects the remote centre of rotation, wherein the second arm is rotatable with respect to the first arm about a second axis intersecting the remote centre of rotation and which is under an angle with, preferably perpendicular to, the first axis. Preferably the ends of the arms extend under an angle and are for instance arc-shaped extending parallel to a spherical surface around the remote centre of rotation and are interconnected at the ends thereof.

To rotate the arms with respect to the stationary part, respectively the other arm, the system further preferably comprises driving means for rotating the arms for aligning the guiding axis of the axial guide with the insertion axis. The driving means are suitably operated such that the axial guide is oriented such that the medical device (to be) held therein is aligned in the predetermined orientation while intersecting the remote centre of rotation. A controller can be provided to automatically control the driving means and/or arms.

As said, according to the invention only a single imaging step is necessary to determine the position and orientation of the stationary part, such that the alignment of the medical device to be held can take place using the determined position and orientation as a reference. In order to align the medical device correctly, also the relative position of the axial guide with respect to the stationary part is necessary. It is for instance possible to determine the position and orientation of the medical device and/or the axial guide during the step of determining the position and orientation the stationary part. The axial guide and/or the medical device can hereto be provided with suitable fiducial markers.

However, a further preferred embodiment of the system further comprises a detector for determining the position and/or orientation of the axial guide with respect to the stationary part, wherein the alignment device is arranged to rotate the axial guide with respect to the stationary part based on the determined position and/or orientation. The detector may for instance include visual detection means arranged to visually detect the relative positions of the parts of the alignment device, for instance the two arms as described above, wherein a processor of for instance the controller is arranged to determine the relative positions of the parts of the alignment device based on the visual detections.

A reliable and efficient detection is further achieved when the detector is formed integrally with the driving means arranged for driving the alignment device. The detector may for instance comprise a digital Magneto-resistant (MR) encoder for detecting the relative movement in the driving means. In addition thereto or alternatively, it is for instance possible that driving means are calibrated such that the exact relative positions of the parts of the alignment device, for instance the stationary part, the axial guide and/or the arms, are known for each magnitude or duration of the driving signal, such that the position with respect to the stationary part can be controlled, for instance by suitable controller, based on the magnitude and/or duration of the driving signal. The system, for instance the controller as discussed below, can hereto be provided with memory means for storing the relative and/or absolute positions of the parts of the alignment device.

In order to increase the accuracy of the alignment device, it is preferred if the relative movement of each of the parts of the alignment device is limited in at least one direction, for instance by an abutment, wherein the relative positions of the parts in this limited position are known.

This position then functions as a reference position. The parts can then be accurately moved from their respective reference positions to their respective positions such that the axial guide is properly positioned for aligning the guiding axis with the insertion axis.

As said, the system further comprises a controller for controlling the operation of the system. The controller preferably is arranged to determine the position and orientation of the stationary part of the alignment device on the basis of the position and orientation of the imaged fiducial marker(s). The controller may for instance receive the exact positions of the preferably a plurality of markers, for instance via a suitable connection to a medical imaging apparatus or a processing device associated therewith. The same applies to the determination of the position of the target location in the patient for the (re)calculation of the insertion axis as mentioned earlier.

On the basis of the position of the markers, the position and orientation of the stationary part can be determined. The controller is hereto provided with suitable processing means, which are as such known in art. It is however also possible that the position and orientation of the stationary part are already determined, for instance by the processing device as mentioned above, and that the position and orientation are provided to the controller.

On the basis of the obtained position and orientation of the stationary part of the alignment device, the controller is further preferably arranged to control the alignment device for aligning the guiding axis of the axial guide with the insertion axis using, preferably exclusively, the position and orientation of the stationary part of the alignment device as a reference. The controller is for instance hereto operatively connected to the driving means of the alignment device for actively aligning the guiding axis.

As the system according to the invention is intended to be used in combination with a medical imaging device which typically comprise a patient support, it is preferred if the system can be rigidly attached to such a patient support. This further fixes the relative position of the system according to the invention with respect to the patient. It is preferred if the base is provided with a suitable connector for connecting the system with the surroundings, for instance the patient support for supporting the patient in the medical imaging device. It may be possible that the base comprises a rail which can be rigidly connected to the surroundings, for instance the patient support. The rail may then allow translational movement of the arm with respect to the patient support and thereby the patient. The arm can thereto be provided with a connector having clamp means for clamping the rail.

According to a preferred embodiment, at least the alignment device and the distal part of the arm are dimensioned for fitting within a bore of a medical imaging apparatus, such a MRI-scanner or a CT-scanner. This allows imaging the fiducial marker(s) for determining the position and orientation of the stationary part and imaging the position of the target location in the patient for determining the insertion axis as mentioned above.

According to a further preferred embodiment, the alignment device and at least the distal part of the arm are manufactured from sufficiently radiolucent material in order not to interfere with the medical imaging process. In particular, the distal parts of the system, that is; the parts near the axial guide, hereto do not contain metal parts. Any metal parts, for instance in the driving means for the alignment device, are located at a proximal location and are interconnected with the necessary part using suitable transfer means from sufficiently radiolucent material.

It is remarked that the remote centre of rotation is a geometrical point defined by the arrangement of the alignment device, in particular the allowed relative movement between the stationary part and the axial guide. The remote centre of rotation therefore does not need to be a physical point. However, in order to allow an efficient placement of the remote centre of rotation on or near the location on the patient, it is preferred if the system according to the invention is provided with visualizing means for visualizing the position of the remote centre of rotation of the alignment device.

As an example, in order to allow an efficient and reliable placement of the stationary part with respect to the patient such that the medical device can be accurately aligned, the system preferably comprises a place holder which indicates the position of the remote centre of rotation. The place holder preferably comprises an engaging surface extending at the same position as the remote centre of rotation, such that placing the engaging surface on the location on the patient ensures that the system is placed with the highest accuracy. The place holder is preferably removably connected such that the place holder can be removed after positioning and is more preferably shaped similar to the medical device. More preferably, the axial guide is arranged to removably hold the medical device such that the medical device can be replaced with the place holder and vice versa.

The invention further relates to a combination of a medical imaging apparatus, such as a MRI-scanner or a CT-scanner, and a system according to the invention.

The invention further relates to a method according to appended claim 13 for aligning a medical device with respect to a location on a patient with an insertion axis using a system according to the invention, wherein the method comprises the step of:
- moving the locking means of the arm to the moveable position;
- positioning the remote centre of rotation of the alignment device on the location on the patient;
- moving the locking means of the arm to the locked position;
- determining the position and orientation of the stationary part of the alignment device by imaging the fiducial marker with a medical imaging apparatus;
- rotating the axial guide such that the guiding axis is aligned with the insertion axis using the alignment device wherein the determined position and orientation of the stationary part of the alignment device is used as a reference.

As said, this allows the alignment of a medical device to be held in the axial guide without the need to iteratively image the medical device to asses whether the current orientation or alignment corresponds to the predetermined orientation as in the prior art. According to preferred embodiment, the step of positioning the remote centre of rotation comprises manually moving the stationary part using an associated handle.

A further preferred embodiment further comprises the steps of determining the position of the remote centre of rotation with respect to the target location in the patient after the step of positioning said remote centre of rotation on the patient, and determining the insertion axis such that the insertion axis intersects the remote centre of rotation and the target location in the patient. As the position of the remote centre of rotation is fixed with respect to the stationary part, the position of the remote centre of rotation can be based on the determination of the already determined position of the stationary part. The insertion axis is then (re)calculated, for instance by a controller as mentioned above, such that this insertion axis intersects the remote centre of rotation and the target location in the patient. This procedure allows compensating for any deviations in the placement of the remote centre of rotation on the location on the patient as for instance preoperatively planned.

The present invention is further illustrated by the following Figures, which show a preferred embodiment of the system according to the invention, and are not intended to limit the scope of the invention in any way, wherein:
- Figure 1 schematically shows the combination of a CT-scanner with an alignment system according to the invention in perspective view;
- Figure 2 schematically shows the alignment system of figure 1 in greater detail in perspective view;
- Figure 3 schematically shows the handle bar of the alignment device;
- Figure 4 schematically shows the positions of the fiducials on the system, and;
- Figures 5 - 8 schematically show the different steps for aligning a medical device with respect to a patient in frontal view.

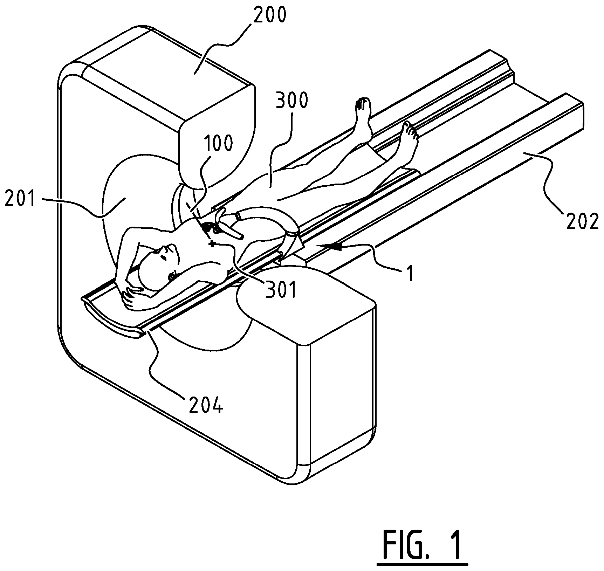

In figure 1, the alignment system 1 according to the invention is shown while being connected to a CT-scanner 200. Such a scanner 200 is typically provided with a bore 201 for accommodating the patient 300 during imaging. The patient 300 hereby lies on a support 203 provided with a rail 204 on a lateral side thereof. The support 203 slides in a track 202 fixed to the scanner 200, and thereby the surroundings, to move the patient 300 in an out of the bore 201.

The system 1 is in particularly useful for interventions where a medical device 100 is to be inserted into the patient 300 at a predetermined incision location 301 while being aligned in a predetermined orientation with an insertion axis. The predetermined location 301 and orientation are preoperatively determined based on medical scans and are for instance chosen such that the medical device 100, when inserted into the patient, damages as little as possible tissue while reaching the target tissue in the patient 300.

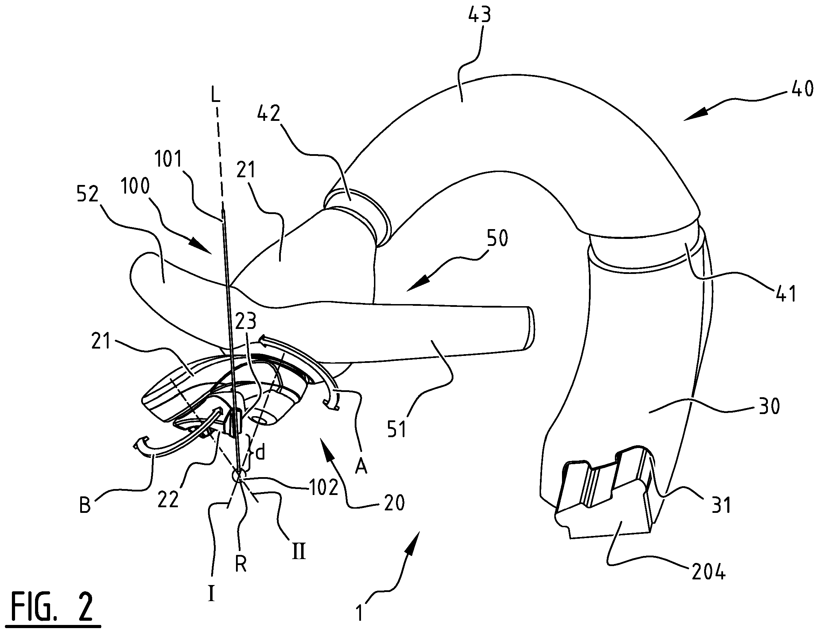

The system 1 according to the invention is arranged for holding and aligning a medical device 100 with respect to the patient 300. With reference to figure 2, the system 1 is hereto provided with a alignment device 20 which is arranged to move an axial guide 23 for guiding the medical device 100 such that the medical device 100 is correctly aligned. More specifically, the alignment device 20 is arranged to rotate the axial guide 23, and thereby the held medical device 100, about a remote centre of rotation R, the position of which being fixed with respect to a stationary part 21 of the alignment device 20. The alignment device 20 is arranged such that the distance d between the remote centre of rotation R and the axial guide 23 is also fixed, regardless of the orientation of the held medical device 100.

The alignment device 20 is hereto provided with a first arc-shaped arm 21 which is rotably connected at a first end with the stationary part 21 of the alignment device 20. The first arm 21 can be rotated in the direction A with respect to the stationary part 21 about a first axis I which intersects the remote centre of rotation R. The other end of the first arm 12 is rotatably connected to a first end of a second arc-shaped arm 22. The axial guide 23 is provided at the other end of the second arm 22. The second arm 22 can be rotated in direction B about a second axis II which also intersects the remote centre of rotation R with respect to the first arm 21. The first and second axes I, II further extend under an angle, in this example perpendicularly. Also the longitudinal axis L of the medical device 100 held in the axial guide extends under an angle with respect to the axes I and II, while at the same time intersecting the remote centre of rotation R. The arms 21, 22 are hereto suitably curved and in this example the curvatures of the arms 21, 22 correspond to the curvature of a spherical surface having the remote centre of rotation R as a centre. This arrangement allows movement of the axial guide 23 along a (virtual) spherical surface while the longitudinal axis L of the medical device 100 intersects the remote centre of rotation R while the distance d remains the same.

The stationary part 21 of the alignment device 20 is connected via an arm 40 to a base 30. At a first end, the arm 40 is provided with a first ball joint 41 connecting the base 30 and an arm section 43. The other end of the arm section 43 is connected via a second ball joint 42 to the part 21 of the alignment device 20. The ball joints 41, 42 function as locking means and are moveable between a moveable position, wherein relative movement of the connected parts is allowed, and a locked position wherein relative movement is locked. The movement between the locked and moveable positions of the ball joints 41, 42 is achieved by a button (not shown) which is located remotely from the system 1. The first ball joint 41 is arranged to allow movement of the arm section 43 with respect to the base 30 in the moveable position, while the second ball joint 32 allows movement between the arm section 42 and the alignment device 20.

The base 30 of the alignment system 1 is connected to the rail 204 using an at least partially complementary to the rail 204 shaped clamp member 31. The clamp member 31 allows a robust and rigid connection of the base 30, and thereby the alignment system 1, to the surroundings.

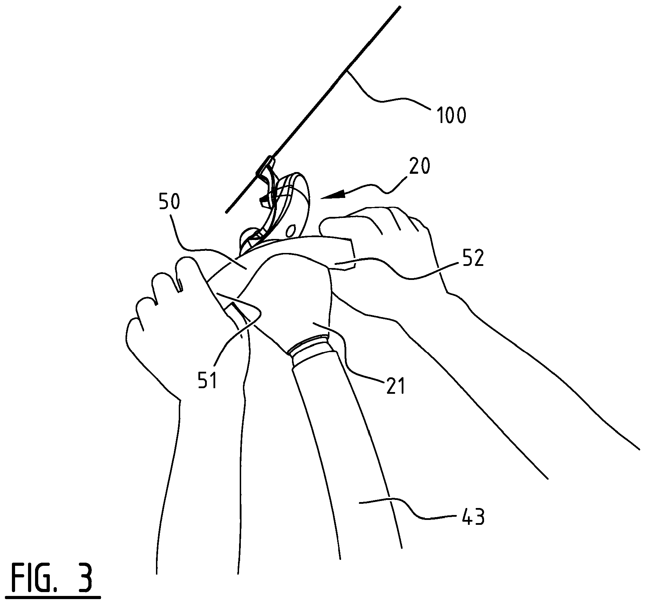

In order to move the stationary part 21 with respect to the patient in the moveable position of the locking means, the stationary part 21 is provided with a handle 50. The handle 50 comprises a first part 51 extending at a first side of the alignment device 20 and a second part 52 extending at the other side of the alignment device 20, such that the alignment device 20 is located in the middle region of the handle 50. This allows the stationary part 21 to be manually moved by gripping both handle parts 51, 52, see figure 3. This allows the user to steer the stationary part 21 to the desired location. More specifically, this allows the remote centre of rotation R, see figure 2, to be positioned on the predetermined location 301 on the patient 300, see figure 1.

To allow an accurate placement, it is possible to use a placeholder 101 provided with an indicator 102 (see again figure 2) which indicates the exact position of the remote centre of rotation R, which is fixed with respect to the stationary part 21. At a later stage, this placeholder 101 can be exchanged with the actual medical device. The indicator 102 can be placed on the predetermined location 301 by steering using the handle 50 and if the indicator is accurately placed, the locking means are moved into the locked position. The placeholder 101 can now be removed, as the position of the remote centre of rotation R is now also fixed with respect to the patient 300.

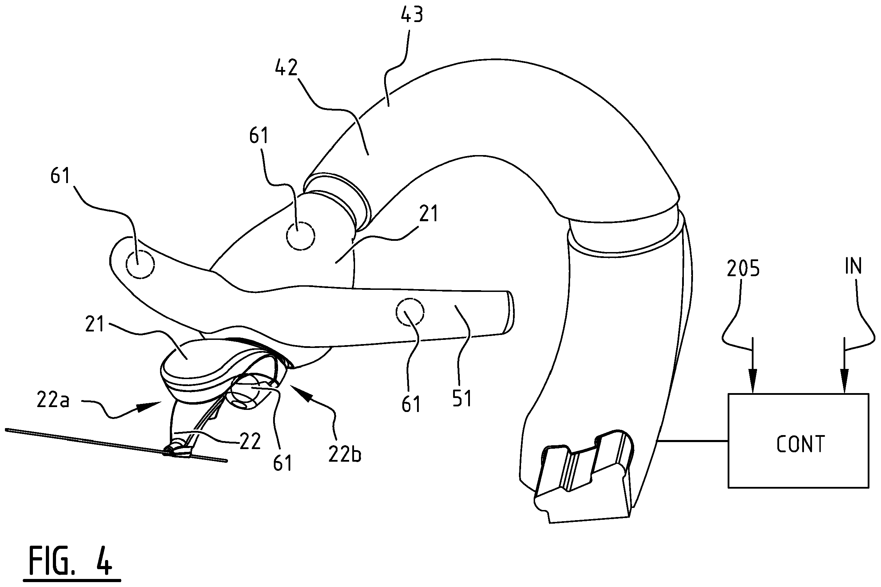

In order to align the medical device 100 in the predetermined orientation, the position and orientation of the stationary part 21 are determined using the CT-scanner 200. With reference to figure 4, the stationary part 21 and the handle 50 are hereto provided with fiducial markers 61 which in combination allows the exact determination of the stationary part 21. In this example, both ends of the handle parts 51, 52 are provided with a fiducial 61. Also the stationary part 21 at locations near the arm section 43 and at the connection with the first arm 21 is provided with fiducials 61.

The position and orientation of the stationary part 21 are determined in a controller, schematically indicated with CONT in figure 4. The controller receives input 205 from the CT-scanner 200 and is arranged to determine the position and orientation of the stationary part 21 based on this input 205. The controller is further operatively coupled to actuators 21a and 21b which are arranged for rotating the arms 21 and 22. The actuators 21a and 21b are operatively coupled using suitable transfers to motors arranged near the base 30 in the system 1, such that these motors do not interfere with the imaging process. The controller CONT is further arranged to only control the actuators 21a and 21b when the locking means are in the locked position.

On the basis of a predetermined orientation of the medical device 100, the input of which into the controller is schematically indicated with IN, the controller operates the actuators 21a and 22a such that the medical device 100 can be aligned in accordance with the predetermined input. The controller hereby uses the position of the stationary part 21 as a reference point, wherein due to the driving of arms 21 and 22, the exact alignment of the medical device 100 can be achieved. In this example, the exact relative position between on the one hand the stationary part 21 and the first arm 21 and on the other hand between the first arm 21 and the second arm 22 is constantly monitored in the actuators 21a and 22a, such that their relative positions are known. It is further preferred if also the motors for driving the actuators are provided with detectors for determining the relative positions of the parts. Also these positions are then used as feedback for driving the actuators 21a and 22a.

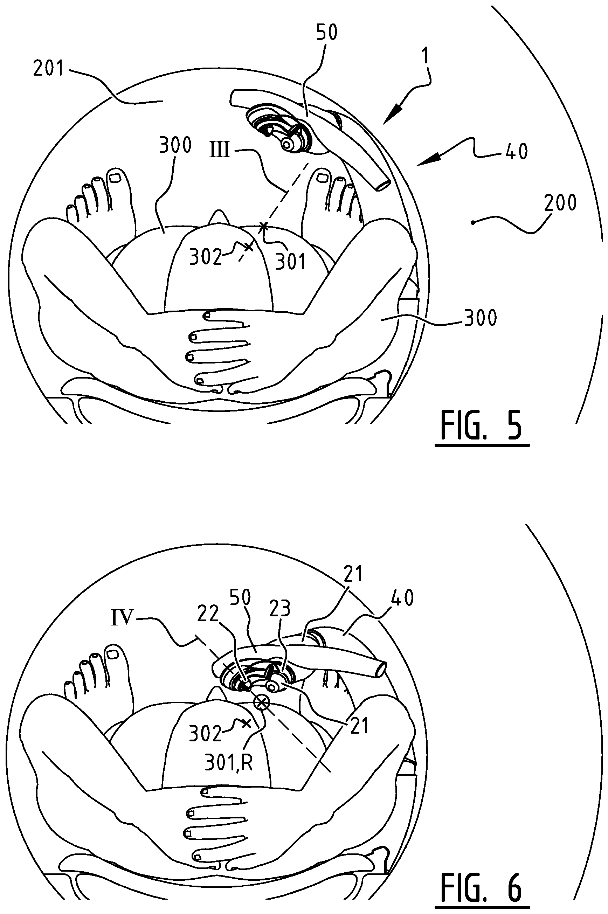

The process of aligning a medical device 100 with respect to a patient 300 is further explained with reference to figures 5 - 8. In a first step, see figure 5, the system 1 is at rest. Preoperatively, an insertion axis III is determined by the physician which allows the insertion of a medical device in the patient to an internal target location 302 with minimal damage to surrounding tissue. Based on this insertion axis III, an optimal insertion location 301 is determined on the patient 300.

The remote centre of rotation R of the alignment device 20 is also indicated. Subsequently, the arm 40 is moved to the moving position, such that by using the handle 50, see figure 3, the remote centre of rotation R can be placed on the insertion location 301. After placement, the arm 40 is moved towards the locked position, such that the remote centre of rotation R with respect to the patient 300 is locked, provided that the patient lies still.

The actual position of the remote centre of rotation R is then checked with respect to the target location 301 by determining the position and orientation of the stationary part 21 of the system 1 using the scanner 200. Based on this actual location, which may differ from the original insertion location 301, the insertion axis III is recalculated by the controller such that the insertion axis III intersects the remote centre of rotation R and the target location 302.

In this example however, the remote centre of rotation R is placed accurately on the insertion location 301 such these locations are shown overlapping in figure 6.

The axial guide 23 as shown in figure 6 is arranged to guide a medical device along a guiding axis IV. This axis IV is fixed with respect to the axial guide 23, such that by rotating the axial guide 23, the guiding axis IV can be aligned with the insertion axis III.

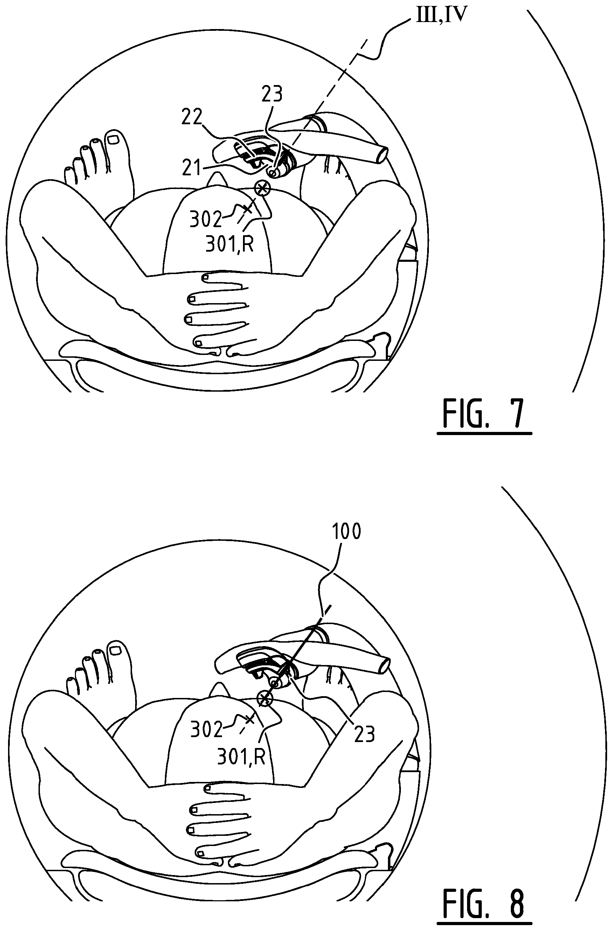

More specifically, using the already determined position of the stationary part 21 as a reference point, the controller moves the arms 21, 22 such that the guiding axis IV of the axial guide 23 aligns with the insertion axis III while the remote centre of rotation R overlaps with the location 301. Reference is made to the differences in orientation of the arms in figures 6 and 7. After correct alignment, the medical device 100 is inserted in the axial guide 23 for the remainder of the procedure, see figure 8.

The present invention is not limited to the embodiment shown, but extends also to other embodiments falling within the scope of the appended claims. The invention is in particular not limited to a particular imaging method.

## Claims

1. System (1) for aligning a medical device (100) with respect to a location (301) on a patient (300) with an insertion axis extending through said location (301) and a predetermined target location (302) in said patient (300), wherein the system comprises:
- a base (30) arranged for connecting the system to the surroundings, for instance a patient support of a scanner;
- an alignment device (20) comprising a stationary part (21) and an axial guide (23), wherein the axial guide (23) is arranged for guiding the medical device (100) along a guiding axis, wherein the alignment device (23) is arranged for rotating the axial guide (23) about a remote centre of rotation (12) while the guiding axis extends through said remote centre of rotation (12);
- an arm (40) connecting the base (30) and the stationary part (21) of the alignment device (20),
wherein the arm (40) comprises locking means which are moveable between a locked position wherein the position of the alignment device (20) with respect to the base (30) is fixed and a moveable position wherein the alignment device (20) is moveable with respect to the base (30) for positioning the remote centre of rotation (12) on the location on the patient (301), wherein the system (1) comprises at least one fiducial marker (61) for determining the position and orientation of the stationary part (21) of the alignment device (20) by an imaging technique, **characterised in that** the alignment device (20) is arranged such that the position of the remote centre of rotation (12) with respect to the stationary part (21) is fixed and further comprising a controller to control the the alignment device (20) to rotate the axial guide (23) about the remote centre of rotation (12) with respect to the stationary part (21) for aligning the guiding axis with the insertion axis using the determined position and orientation of said stationary part (21) as a reference.

2. System (1) according to claim 1, wherein the system (1) comprises a handle (50) associated with the stationary part (21) of the alignment device (20) and wherein the locking means are arranged to allow manual movement between the base (30) and the stationary part (21) in the moveable position for manually positioning the remote centre of rotation (12) on the location on the patient (300).

3. System (1) according to claim 2, wherein the handle (50) comprises at least one fiducial marker (61).

4. System (1) according to any of the preceding claims, further comprising the controller arranged for determining the position of the remote centre of rotation (12) on the patient (300) based on the determined position and orientation of the stationary part (21), wherein the controller is further arranged to determine the insertion axis based on the position of the target location (302) in the patient (300) and the position of the remote centre of rotation (12) such that the insertion axis intersect the remote centre of rotation (12) and the target location (302) in the patient (300).

5. System (1) according to any of the preceding claims, wherein the arm (40) comprises a plurality of arm sections (43) interconnected by lockable ball joints (41, 42).

6. System (1) according to any of the preceding claims, wherein the alignment device (20) comprises a first arm (21) and a second arm (22) comprising the axial guide (23), wherein the first arm (21) is rotatably connected to the stationary part (21), wherein the first arm (21) is rotatable with respect to the stationary part (21) and wherein the second arm (22) is rotatable with respect to the first arm (21) for rotating the axial guide (23) about the remote centre of rotation (12) along at least part of a spherical surface, wherein the system further comprises driving means for rotating the arms (21, 22) for aligning the guiding axis with the insertion axis.

7. System (1) according to any of the preceding claims, wherein the system (1) comprises a detector for determining the position and orientation of the axial guide (23) with respect to the stationary part (21), wherein the alignment device (20) is arranged to rotate the axial guide (23) with respect to the stationary part (21) based on the determined position and orientation, wherein the detector is preferably formed integrally with the driving means arranged for driving the alignment device (20).

8. System (1) according to any of the preceding claims, wherein the controller is arranged to determine the position and orientation of the stationary part (21) of the alignment device (20) on the basis of the position and orientation of the imaged fiducial marker (61), wherein the controller is arranged to control the alignment device for aligning the guiding axis of the axial guide with the insertion axis using the position and orientation of the stationary part (21) of the alignment device (20) as a reference.

9. System (1) according to claim 8, wherein the controller is arranged to control the alignment device for aligning the guiding axis of the axial guide with the insertion axis using exclusively the position and orientation of the stationary part (21) of the alignment device (20) as a reference.

10. System (10) according to any of the preceding claims, wherein at least the alignment device (20) and the distal part (40) of the arm are dimensioned for fitting within a bore of a medical imaging apparatus, such a MRI-scanner or a CT-scanner, and are manufactured from sufficiently radiolucent material in order not to interfere with the medical imaging process.

11. System (1) according to any of the preceding claims, further comprising visualizing means (100) for visualizing the position of the remote centre of rotation (12) of the alignment device (20).

12. Combination of a medical imaging apparatus (200), such as a MRI-scanner or a CT-scanner, and a system (1) according to any of the preceding claims.

13. Method for aligning a medical device (100) with respect to a location (301) on a patient (300) with an insertion axis using a system (1) according to any of the preceding claims, wherein the method comprises the step of:
- moving the locking means of the arm (40) to the moveable position;
- positioning the remote centre of rotation (12) of the alignment device (20) on the position (301) on the patient (300);
- moving the locking means of the arm (40) to the locked position;
- determining the position and orientation of the stationary part (21) of the alignment device (20) by imaging the fiducial marker (61) with a medical imaging apparatus (200);
- rotating the axial guide (23) such that the guiding axis is aligned with the insertion axis using the alignment device (20) wherein the determined position and orientation of the stationary part (21) of the alignment device (20) is used as a reference.

14. Method according to claim 13, wherein the step of positioning the remote centre of rotation (12) comprises manually moving the stationary part (21) using an associated handle.

15. Method according to claim 13 or 14, further comprising the steps of determining the position of the remote centre of rotation (12) with respect to the target location (301) in the patient (300) after the step of positioning said remote centre of rotation (12) on the patient (301) and determining the insertion axis such that the insertion axis intersects the remote centre of rotation and the target location (302) in the patient (300).

## Patentansprüche

1. System (1) zur Ausrichtung einer medizinischen Vorrichtung (100) in Bezug auf einen Ort auf einem Patienten (300) mit einer Einsetzachse, die sich durch den Ort (301), und einen vorbestimmten Zielort (302) in dem Patienten (300) erstreckt, wobei das System aufweist:
- eine Basis (30), die eingerichtet ist, um das System mit der Umgebung, zum Beispiel einer Patientenauflage eines Scanners, zu verbinden;
- eine Ausrichtungsvorrichtung (20), die einen ortsfesten Teil (21) und eine Axialführung (23) aufweist, wobei die Axialführung (23) eingerichtet ist, um die medizinische Vorrichtung (100) entlang einer Führungsachse zu führen, wobei die Ausrichtungsvorrichtung (23) eingerichtet ist, um die Axialführung (23) um ein entferntes Drehzentrum (12) zu drehen, während die Führungsachse sich durch das entfernte Drehzentrum (12) erstreckt;
- einen Arm (40), der die Basis (30) und den ortsfesten Teil (21) der Ausrichtungsvorrichtung (20) verbindet,
wobei der Arm (40) Sperreinrichtungen aufweist, die zwischen einer gesperrten Position, in der die Position der Ausrichtungsvorrichtung (20) in Bezug auf die Basis (30) fixiert ist, und einer beweglichen Position, in der die Ausrichtungsvorrichtung (20) in Bezug auf die Basis (30) beweglich ist, um das entfernte Drehzentrum (12) auf dem Ort des Patienten (301) zu positionieren, beweglich ist, wobei das System (1) wenigstens eine Bezugsmarke (61) aufweist, um die Position und Orientierung des ortsfesten Teils (21) der Ausrichtungsvorrichtung (20) durch ein Abbildungsverfahren zu bestimmen, **dadurch gekennzeichnet, dass** die Ausrichtungsvorrichtung (20) derart eingerichtet ist, dass die Position des entfernten Drehzentrums (12) in Bezug auf den ortsfesten Teil (21) fixiert ist, und sie ferner eine Steuerung aufweist, um die Ausrichtungsvorrichtung (20) zu steuern, um die Axialführung (23) in Bezug auf den ortsfesten Teil (21) um das entfernte Drehzentrum (12) zu drehen, um die Führungsachse mit der Einsetzachse unter Verwendung der bestimmten Position und Orientierung des ortsfesten Teils (21) als eine Referenz auszurichten.

2. System (1) nach Anspruch 1, wobei das System (1) einen Griff (50) aufweist, der zu dem ortsfesten Teil (21) der Ausrichtungsvorrichtung (20) gehört, und wobei die Sperreinrichtungen eingerichtet sind, um die manuelle Bewegung zwischen der Basis (30) und dem ortsfesten Teil (21) in der beweglichen Position zuzulassen, um das entfernte Drehzentrum (12) auf dem Ort auf dem Patienten manuell (300) zu positionieren.

3. System (1) nach Anspruch 2, wobei der Griff (50) wenigstens eine Bezugsmarke (61) aufweist.

4. System (1) nach einem der vorhergehenden Ansprüche, das ferner die Steuerung aufweist, die eingerichtet ist, um die Position des entfernten Drehzentrums (12) auf dem Patienten (300) basierend auf der bestimmten Position und Orientierung des ortsfesten Teils (21) zu bestimmen, wobei die Steuerung ferner eingerichtet ist, um die Einsetzachse basierend auf der Position des Zielorts (302) in dem Patienten (300) und der Position des entfernten Drehzentrums (12) derart zu bestimmen, dass die Einsetzachse das entfernte Drehzentrum (12) und die Zielstelle (302) in dem Patienten (300) schneidet.

5. System (1) nach einem der vorhergehenden Ansprüche, wobei der Arm (40) mehrere Armabschnitte (43) aufweist, die durch sperrbare Kugelgelenke (41, 42) miteinander verbunden sind.

6. System (1) nach einem der vorhergehenden Ansprüche, wobei die Ausrichtungsvorrichtung (20) einen ersten Arm (21) und einen zweiten Arm (22) der die Axialführung (20) aufweist, aufweist, wobei der erste Arm (21) drehbar mit dem ortsfesten Teil (21) verbunden ist, wobei der erste Arm (21) in Bezug auf den ortsfesten Teil (21) drehbar ist und wobei der zweite Arm (22) in Bezug auf den ersten Arm (21) drehbar ist, um die Axialführung (23) um die entfernte Drehmitte (12) entlang wenigstens eines Teils einer kugelförmigen Oberfläche zu drehen, wobei das System ferner Antriebseinrichtungen zum Drehen der Arme (21, 22) aufweist, um die Führungsachse mit der Einsetzachse auszurichten.

7. System (1) nach einem der vorhergehenden Ansprüche, wobei das System (1) einen Detektor zur Bestimmung der Position und Orientierung der Axialführung (23) in Bezug auf den ortsfesten Teil (21) aufweist, wobei die Ausrichtungsvorrichtung (20) eingerichtet ist, um die Axialführung (23) in Bezug auf den ortsfesten Teil (21) basierend auf der bestimmten Position und Orientierung zu drehen, wobei der Detektor bevorzugt integral mit den Antriebseinrichtungen ausgebildet ist, die zum Antreiben der Ausrichtungsvorrichtung (20) eingerichtet sind.

8. System (1) nach einem der vorhergehenden Ansprüche,
wobei die Steuerung eingerichtet ist, um die Position und Orientierung des ortsfesten Teils (21) der Ausrichtungsvorrichtung (20) auf der Basis der Position und Orientierung der abgebildeten Bezugsmarke (61) zu bestimmen, wobei die Steuerung eingerichtet ist, um die Ausrichtungsvorrichtung zur Ausrichtung der Führungsachse der Axialführung mit der Einsetzachse unter Verwendung der Position und Orientierung des ortsfesten Teils (21) der Ausrichtungsvorrichtung (20) als ein Bezug zu steuern.

9. System (1) nach Anspruch 8, wobei die Steuerung eingerichtet ist, um die Ausrichtungsvorrichtung zur Ausrichtung der Führungsachse der Axialführung mit der Einsetzachse ausschließlich unter Verwendung der Position und Orientierung des ortsfesten Teils (21) der Ausrichtungsvorrichtung (20) als ein Bezug zu steuern.

10. System (10) nach einem der vorhergehenden Ansprüche, wobei wenigstens die Ausrichtungsvorrichtung (20) und der distale Teil (40) des Arms bemessen sind, um in eine Bohrung einer medizinischen Abbildungsvorrichtung, wie etwa eines MRI-Scanners oder eines CT-Scanners zu passen, und aus ausreichend strahlendurchlässigem Material hergestellt sind, um das medizinische Abbildungsverfahren nicht zu stören.

11. System (1) nach einem der vorhergehenden Ansprüche, das ferner Visualisierungseinrichtungen (100) zur Visualisierung der Position des entfernten Drehzentrums (12) für die Ausrichtungsvorrichtung (20) aufweist.

12. Kombination einer medizinischen Abbildungsvorrichtung (200), wie etwa eines MRI-Scanners oder eines CT-Scanners, und eines Systems (1) nach einem der vorhergehenden Ansprüche.

13. Verfahren zur Ausrichtung einer medizinischen Vorrichtung (100) in Bezug auf einen Ort (301) auf einem Patienten (300) mit einer Einsetzachse unter Verwendung eines Systems (1) nach einem der vorhergehenden Ansprüche, wobei das Verfahren die folgenden Schritte aufweist:
- Bewegen der Sperreinrichtungen des Arms (40) zu der beweglichen Position;
- Positionieren des entfernten Drehzentrums (12) der Ausrichtungsvorrichtung (20) auf der Position (301) auf dem Patienten (300);
- Bewegen der Sperreinrichtungen des Arms (40) in die gesperrte Position;
- Bestimmen der Position und Orientierung des ortsfesten Teils (21) der Ausrichtungsvorrichtung (20) durch Abbilden der Bezugsmarke (61) mit einer medizinischen Abbildungsvorrichtung (200);
- Drehen der Axialführung (23) derart, dass die Führungsachse unter Verwendung der Ausrichtungsvorrichtung (20) mit der Einsetzachse ausgerichtet wird, wobei die bestimmte Position und Orientierung des ortsfesten Teils (21) der Ausrichtungsvorrichtung (20) als eine Referenz verwendet werden.

14. Verfahren nach Anspruch 13, wobei der Schritt der Positionierung des entfernten Drehzentrums (12) das manuelle Bewegen des ortsfesten Teils (21) unter Verwendung eines zugehörigen Griffs aufweist.

15. Verfahren nach Anspruch 13 oder 14, das ferner die Schritte der Bestimmung der Position des entfernten Drehzentrums (12) in Bezug auf den Zielort (301) in dem Patienten (300) nach dem Schritt des Positionierens des entfernten Drehzentrums (12) auf dem Patienten (301) und Bestimmen der Einsetzachse derart, dass die Einsetzachse das entfernte Drehzentrum und den Zielort (302) in dem Patienten (300) schneidet, aufweist.

## Revendications

1. Système (1) d'alignement d'un dispositif médical (100) par rapport à un site (301) sur un patient (300) avec un axe d'insertion s'étendant à travers ledit site (301) et un site cible (302) prédéterminé chez ledit patient (300), dans lequel le système comprend :
- une base (30) agencée pour relier le système à l'environnement, par exemple un support pour patient d'un scanner ;
- un dispositif d'alignement (20) comprenant une partie statique (21) et un guide axial (23), dans lequel le guide axial (23) est agencé pour guider le dispositif médical (100) le long d'un axe de guidage, dans lequel le dispositif d'alignement (23) est agencé pour faire tourner le guide axial (23) autour d'un centre de rotation distant (12) tandis que l'axe de guidage s'étend à travers ledit centre de rotation distant (12) ;
- un bras (40) reliant la base (30) et la partie statique (21) du dispositif d'alignement (20),
dans lequel le bras (40) comprend des moyens de verrouillage qui sont mobiles entre une position verrouillée dans laquelle la position du dispositif d'alignement (20) par rapport à la base (30) est fixe, et une position mobile dans laquelle le dispositif d'alignement (20) est mobile par rapport à la base (30) pour positionner le centre de rotation distant (12) sur le site sur le patient (301), dans lequel le système (1) comprend au moins un marqueur de repère (61) pour déterminer la position et l'orientation de la partie statique (21) du dispositif d'alignement (20) par une technique d'imagerie, **caractérisé en ce que** le dispositif d'alignement (20) est agencé de sorte que la position du centre de rotation distant (12) par rapport à la partie statique (21) soit fixe, et comprenant en outre un dispositif de commande pour commander le dispositif d'alignement (20) pour faire tourner le guide axial (23) autour du centre de rotation distant (12) par rapport à la partie statique (21) pour aligner l'axe de guidage avec l'axe d'insertion en utilisant la position et l'orientation déterminées de ladite partie statique (21) comme référence.

2. Système (1) selon la revendication 1, dans lequel le système (1) comprend une poignée (50) associée à la partie statique (21) du dispositif d'alignement (20) et dans lequel les moyens de verrouillage sont agencés pour permettre un déplacement manuel entre la base (30) et la partie statique (21) dans la position mobile pour positionner manuellement le centre de rotation distant (12) sur le site sur le patient (300).

3. Système (1) selon la revendication 2, dans lequel la poignée (50) comprend au moins un marqueur de repère (61).

4. Système (1) selon l'une quelconque des revendications précédentes, comprenant en outre le dispositif de commande agencé pour déterminer la position du centre de rotation distant (12) sur le patient (300) sur la base de la position et de l'orientation déterminées de la partie statique (21), dans lequel le dispositif de commande est en outre agencé pour déterminer l'axe d'insertion sur la base de la position du site cible (302) sur le patient (300) et de la position du centre de rotation distant (12) de sorte que l'axe d'insertion coupe le centre de rotation distant (12) et le site cible (302) chez le patient (300) .

5. Système (1) selon l'une quelconque des revendications précédentes, dans lequel le bras (40) comprend une pluralité de sections de bras (43) interconnectées par des articulations à rotule verrouillables (41, 42).

6. Système (1) selon l'une quelconque des revendications précédentes, dans lequel le dispositif d'alignement (20) comprend un premier bras (21) et un second bras (22) comprenant le guide axial (23), dans lequel le premier bras (21) est relié de manière rotative à la partie statique (21), dans lequel le premier bras (21) peut tourner par rapport à la partie statique (21) et dans lequel le second bras (22) peut tourner par rapport au premier bras (21) pour faire tourner le guide axial (23) autour du centre de rotation distant (12) le long d'au moins une partie d'une surface sphérique, dans lequel le système comprend en outre des moyens d'entraînement pour faire tourner les bras (21, 22) pour aligner l'axe de guidage avec l'axe d'insertion.

7. Système (1) selon l'une quelconque des revendications précédentes, dans lequel le système (1) comprend un détecteur pour déterminer la position et l'orientation du guide axial (23) par rapport à la partie statique (21), dans lequel le dispositif d'alignement (20) est agencé pour faire tourner le guide axial (23) par rapport à la partie statique (21) sur la base de la position et de l'orientation déterminées, dans lequel le détecteur est de préférence formé d'un seul tenant avec les moyens d'entraînement agencés pour entraîner le dispositif d'alignement (20).

8. Système (1) selon l'une quelconque des revendications précédentes, dans lequel le dispositif de commande est agencé pour déterminer la position et l'orientation de la partie statique (21) du dispositif d'alignement (20) sur la base de la position et de l'orientation du marqueur de repère (61) imagé, dans lequel le dispositif de commande est agencé pour commander le dispositif d'alignement pour aligner l'axe de guidage du guide axial avec l'axe d'insertion en utilisant la position et l'orientation de la partie statique (21) du dispositif d'alignement (20) comme référence.

9. Système (1) selon la revendication 8, dans lequel le dispositif de commande est agencé pour commander le dispositif d'alignement pour aligner l'axe de guidage du guide axial avec l'axe d'insertion en utilisant exclusivement la position et l'orientation de la partie statique (21) du dispositif d'alignement (20) comme référence.

10. Système (10) selon l'une quelconque des revendications précédentes, dans lequel au moins le dispositif d'alignement (20) et la partie distale (40) du bras sont dimensionnés pour s'ajuster à l'intérieur d'un alésage d'un appareil d'imagerie médicale, tel qu'un scanner IRM ou un scanner CT, et sont fabriqués à partir d'un matériau suffisamment radiotransparent pour ne pas interférer avec le processus d'imagerie médicale.

11. Système (1) selon l'une quelconque des revendications précédentes, comprenant en outre des moyens de visualisation (100) pour visualiser la position du centre de rotation distant (12) du dispositif d'alignement (20).

12. Combinaison d'un appareil d'imagerie médicale (200), tel qu'un scanner IRM ou un scanner CT, et d'un système (1) selon l'une quelconque des revendications précédentes.

13. Procédé d'alignement d'un dispositif médical (100) par rapport à un site (301) sur un patient (300) avec un axe d'insertion en utilisant un système (1) selon l'une quelconque des revendications précédentes, dans lequel le procédé comprend les étapes de :
- déplacement des moyens de verrouillage du bras (40) vers la position mobile ;
- positionnement du centre de rotation distant (12) du dispositif d'alignement (20) sur la position (301) sur le patient (300) ;
- déplacement des moyens de verrouillage du bras (40) vers la position verrouillée ;
- détermination de la position et de l'orientation de la partie statique (21) du dispositif d'alignement (20) en imaginant le marqueur de repère (61) avec un appareil d'imagerie médicale (200) ;
- rotation du guide axial (23) de sorte que l'axe de guidage soit aligné avec l'axe d'insertion en utilisant le dispositif d'alignement (20), dans lequel la position et l'orientation déterminées de la partie statique (21) du dispositif d'alignement (20) sont utilisées comme référence.

14. Procédé selon la revendication 13, dans lequel l'étape de positionnement du centre de rotation distant (12) comprend le déplacement manuel de la partie statique (21) en utilisant une poignée associée.

15. Procédé selon la revendication 13 ou 14, comprenant en outre les étapes de détermination de la position du centre de rotation distant (12) par rapport au site (301) cible chez le patient (300) après l'étape de positionnement dudit centre de rotation distant (12) sur le patient (301), et de détermination de l'axe d'insertion de sorte que l'axe d'insertion coupe le centre de rotation distant et le site cible (302) chez le patient (300).
